# EUROPÄISCHE PATENTSCHRIFT

(11) **EP 3 720 371 B1**
(45) Veröffentlichungstag und Bekanntmachung des Hinweises auf die Patenterteilung: **08.09.2021**
(21) Anmeldenummer: 19702812.9
(22) Anmeldetag: 18.01.2019
(51) Int. Cl.: A61B 17/22, A61B 90/98

(54) **SYSTEM ZUM ZERTRÜMMERN UND/ODER ENTFERNEN VON KÖRPERSTEINEN, VERFAHREN ZUM BEREITSTELLEN EINES DERARTIGEN SYSTEMS**
SYSTEM FOR CRUSHING AND/OR REMOVING BODY STONES, METHOD FOR PROVIDING SUCH A SYSTEM
SYSTÈME POUR LA DESTRUCTION ET/OU L'ÉLIMINATION DE CALCULS CORPORELS, PROCÉDÉ POUR LA FOURNITURE D'UN TEL SYSTÈME

(30) Priorität: 19.01.2018 DE 102018101221
(43) Veröffentlichungstag der Anmeldung: 14.10.2020
(73) Patentinhaber: Ferton Holding S.A., 2800 Delémont (CH)
(72) Erfinder: GATINEAU, Vincent, 74160 St. Julien en Genevois (FR); GIROD, Jean-Yves, 1196 Gland (CH); LONGONI, Paolo, 1296 Coppet (CH); LOK, Maxime, 01170 Échenevex (FR); MICHAUD, Federic, 39400 Morbier (FR); NINI, Gaël, 1010 Lausanne (CH); POULAT, Nicolas, 74600 Seynod (FR); RAMBAUD, Denis, 1205 Genève (CH)
(74) Vertreter: Müller Schupfner & Partner Patent- und Rechtsanwaltspartnerschaft mbB
(86) Internationale Anmeldenummer: PCT/EP2019/051279
(87) Internationale Veröffentlichungsnummer: WO 2019/141821

(56) Entgegenhaltungen:
- EP-A1- 0 676 175
- EP-A2- 0 947 171
- US-A1- 2007 038 160
- US-A1- 2011 089 248
- US-A1- 2011 208 206

## Beschreibung

Die vorliegende Erfindung betrifft ein System zum Zertrümmern und/oder Entfernen von Körpersteinen, ein Verfahren zum Bereitstellen eines derartigen Systems und ein Adapterelement.

Zum Zertrümmern von Körpersteinen, wie z. B. Harnsteinen bzw. Nierensteinen, sind Systeme bekannt, mit denen sich diese zertrümmern bzw. zerkleinern und anschließend so entstandene Bruchstücke der Körpersteine entfernen lassen. Insbesondere handelt es sich dabei um sogenannte intra-korporale Lithotripter, bei denen ein Instrument endoskopisch bis an den Stein herangeführt wird um ihn dann damit zu zertrümmern. Bekannt sind optische Systeme mit einem Laser als Quelle, elektrohydraulische Funkenentladungsgeräte und kontinuierlich oder impulsförmig angeregte Sonden, die die Steine mechanisch zerkleinern. Bei den mechanisch arbeitenden Systemen besteht ein wesentlicher Bestandteil aus einer Stoßwellen und/oder Schwingungen veranlassenden Quelle und einer lösbar mit der Quelle verbundene Sonde. Dabei wird typischerweise die im Wesentlichen nadelförmige oder stangenförmige bzw. hohlzylindrische Sonde mit einem ersten proximalem Ende auf den zu zerstörenden Körperstein ausgerichtet, während über ein dem ersten Ende gegenüberliegendes zweites distales Ende die von der Quelle ausgehenden Stoßwelle und/oder Schwingungen auf die Sonde übertragen wird. Die Sonde leitet bzw. führt dann die am zweiten Ende eingeleitete Stoß- und/oder Schallwelle bis zu ihrem Einsatzgebiet in der Nähe des Körpersteins weiter und zerstört diesen dann durch die resultierende mechanische Bewegung der proximalen Sondenspitze.

Wegen der im Betrieb auftretenden hohen mechanischen Belastungen, insbesondere durch die häufig im Ultraschallbereich schwingenden Sonden, , müssen diesen nach einer begrenzen Anzahl von Einsätzen ausgetauscht werden. Daher sind die Sonden in der Regel über eine Schnittstelle lösbar mit der Quelle verbunden und lassen sich so regelmäßig austauschen. Zudem gestattet es ein im Schnittstellenbereich zu findender Kopplungsmechanismus, dass verschiedene Typen von Sonden an dieselbe Quelle angebunden werden können.

Aus der US 2011 / 0 208 206 A1 ist ein System bekannt, bei dem in die Sonde ein RFID-Sender eingelassen ist. Mittels der Sonde können mittels drahtloser Kommunikation Informationen über die Sonde zu deren Identifikation übermittelt werden. Allerdings hat sich herausgestellt, dass wegen der intensiven Belastung durch Ultraschallschwingungen und durch die sterilisationsbedingten hohen Temperaturen und aggressiven Chemikalien die Funktionalität des RFID-Senders, insbesondere auf Dauer, nachteilhaft beeinträchtigt wird.

Aus der US 2007/0038160 A1 ist ein System zum Zertrümmern von Körpersteinen bekannt, wobei das System einen RFID-Leser aufweist. Das Dokument US 2011/0089248 A1 befasst sich mit einem Schneidezubehör mit einem angetriebenen chirurgischen Werkzeug, das einen RFID-Chip aufweist.

Ausgehend von diesem Hintergrund macht es sich die vorliegende Erfindung zur Aufgabe, ein System zum Zertrümmern und/oder Entfernen von Körpersteinen bereitzustellen, mit dem die Funktionalität des RFID-Elements, insbesondere auch nach mehrmaligem Verbinden der Sonde mit der Quelle, also dauerhaft, sichergestellt werden kann.

Diese Aufgabe wird gelöst durch ein System gemäß Anspruch 1, durch ein Verfahren gemäß Anspruch 9. Weitere Vorteile und Merkmale der Erfindung ergeben sich aus den Unteransprüchen sowie der Beschreibung und der beigefügten Figur.

Erfindungsgemäß ist ein System zum Zertrümmern und/oder Entfernen von Körpersteinen vorgesehen, umfassend:
- eine Stoßwellen und/oder Ultraschallwellen veranlassende Quelle und
- eine Sonde,
wobei die Quelle und die Sonde über eine Schnittstelle zur Übertragung der Stoßwellen und/oder Ultraschallwellen auf die Sonde reversibel verbindbar sind und die Sonde ein Ident-Element zur Identifikation der Sonde und/oder zur Speicherung von Daten umfasst, wobei das Ident-Element schwingungs- und/oder temperaturgeschützt in oder an der Sonde angeordnet ist, so dass das Ident-Element von der Sonde entkoppelt ist.

Das Ident-Element ist dabei bevorzugt ein RFID-Element, kann aber auch ein anderes elektrisches oder elektromagnetisches Element sein. Im Folgenden wird bevorzugt ein RFID-Element genannt, ohne dass die Ausführungsformen auf ein solches Element beschränkt werden.

Gegenüber den aus dem Stand der Technik bekannten Systemen erweist sich das erfindungsgemäße System insbesondere deshalb als vorteilhaft, weil das Ident- bzw. RFID-Element weniger durch die Ultraschallschwingen beeinträchtigt wird. Unter schallgeschützt ist insbesondere zu verstehen, dass die im Betrieb entstehenden Schwingungen, insb. Ultraschallschwingungen nur partiell, d. h. anteilig, auf das RFID-Element übertragen werden. Insbesondere kann als Referenz hierzu verwendet werden, dass mit dem erfindungsgemäßen System die (aufgenommenen) Schwingungen des RFID-Elements reduziert sind gegenüber einem System, bei dem das RFID-Element starr mit der Sonde verbunden ist. Das RFID-Element ist somit schwingungstechnisch von der Sonde entkoppelt. Dabei ist es beispielsweise vorgesehen, dass das RFID-Element für eine schwingungsgeschützte Anordnung locker oder mittelbar über ein Dämpfungsglied mit der Sonde verbunden ist bzw. an dieser angeordnet ist.

Grundsätzlich ist unter einem Ident-Element ein solches Bauteil zu verstehen, dass zur automatischen und berührungslosen Identifikation oder Lokalisierung von Objekten verwendet werden kann. Insbesondere umfasst das Ident-Element einen Transponder, in dem Informationen zu der jeweiligen Sonde hinterlegt werden. Eine Kopplung mit dem Ident-Element erfolgt mittels eines Lesegeräts, das ein magnetisches Wechselfeld mit vergleichsweise geringer Reichweite oder hochfrequente Radiowellen erzeugt. Damit werden nicht nur Daten übertragen, sondern auch der Transponder mit Energie versorgt. Zur Erreichung größerer Reichweiten ist es vorstellbar, das Ident-Element mit einer eigenen Stromversorgung oder einer elektromagnetischen Schnittstelle auszustatten.

Weiterhin ist es vorgesehen, dass die Schnittstelle für das lösbare Verbinden der Quelle und der Sonde über einen entsprechenden Kopplungsmechanismus verfügt. Insbesondere umfasst der Kopplungsmechanismus eine quellseitige Komponente und eine sondenseitige Komponente, die komplementär zueinander ausgestaltet sind und so vorzugsweise miteinander eine formschlüssige und/oder kraftschlüssige Verbindung im verbunden Zustand eingehen. Beispielsweise handelt es sich bei dem Kopplungsmechanismus um ein Gewinde mit einem Innen- und Außengewinde als quellseitige und sondenseitige Komponenten oder einen Bajonettverschluss. Vorzugsweise umfasst das Ident- bzw. RFID-Element eine Speichereinrichtung, in der Informationen über die Sonde hinterlegt werden können. Beispielsweise sind Informationen zum Hersteller, einer Dimensionierung der Sonde, Anzahl der Einsätze der Sonde oder Ähnliches in der Speichereinrichtung gespeichert. Dadurch kann beispielsweise mit Vorteil verhindert werden, dass beispielsweise wegen einer ungeeigneten oder verbrauchten Sonde das System unsachgemäß verwendet wird. Vorzugsweise lässt sich die Speichereinrichtung überschreiben, d. h. es lassen sich auf der Speichereinrichtung gespeicherte Informationen aktualisieren.

Insbesondere handelt es sich bei dem System um einen intra-korporalen Lithotripter, bei dem die Sonde vorzugsweise im Wesentlichen nadelförmig, stangenförmig oder hohlzylindrisch ausgestaltet ist, d. h. die Sonde hat einen im Wesentlichen in einer senkrecht zu ihrer Längsrichtung verlaufenden Ebene kreisförmigen Querschnitt und erstreckt sich in ihrer Längsrichtung gesehen, um ein Vielfaches weiter als ihr in den Querschnittsebene bemessener Durchmesser. Im betriebsbereiten Zustand, d. h. in einem Zustand, in dem die Sonde an die Quelle angebunden ist, ist ein erstes proximales Ende der Sonde von der Quelle abgewandt und das dem ersten Ende gegenüberliegenden zweite distale Ende der Quelle zugewandt. Dadurch lassen sich die Stoßwellen bzw. Schallwellen entlang einer Zentralrichtung von der Quelle auf das zweite Ende der Sonde übertragen und vom zweiten Ende an das erste Ende weiterleiten. Zum Zertrümmern wird dann das erste Ende direkt an dem Stein platziert.

Vorzugsweise ist die Sonde hohl ausgestaltet. Dadurch lässt sich mit Vorteil ein Hohlbereich bereitstellen, über den zerkleinerte Teile bzw. Trümmerteile vom ersten Ende zum zweiten Ende abgeführt z. B. abgesaugt werden können. Ferner ist es vorzugsweise vorgesehen, dass die Sonde an einem Handgerät, in das die Quelle integriert ist, lösbar verbindbar ist. Vorzugsweise wird die Sonde an eine Stirnseite des Handgeräts angebunden. Das Handgerät umfasst dabei vorzugsweise einen Griffbereich und einen Projektilbereich. Im Projektilbereich weist die Quelle vorzugsweise eine erste Teilquelle zur Veranlassung einer Stoßwelle mittels eines Projektils und eine zweite Teilquelle zur Veranlassung einer Schallwelle mittels Piezoelementen auf, die entsprechend eine Stoßwelle und/oder eine Schallwelle in die Sonde einleiten. Mittels des Griffbereichs lässt sich die Sonde vorzugsweise ausrichten bzw. ein Nutzer kann das erste Ende über den Griffbereich ausrichten bzw. positionieren.

Weiterhin ist es vorgesehen, dass die Sonde einen im Wesentlichen die nadelförmige Form der Sonde bestimmenden Hohlkörper und an ihrem zweiten distalen Ende ein Adapterelement zum Anbinden der Sonde an die Quelle aufweist. Im betriebsbereiten Zustand ist eine erste Aussparung des Adapterelements derart auf die Quelle gerichtet, dass das Projektil in die erste Aussparung eingreifen kann. Das Adapterelement und/oder der Hohlkörper ist insbesondere aus einem Metall gefertigt, beispielsweise einem Chrom und/oder Nickel umfassenden Edelstahl.

Gemäß einer bevorzugten Ausführungsform der vorliegenden Erfindung ist es vorgesehen, dass das RFID-Element in ein Ringelement, insbesondere in ein aus Kunststoff gefertigtes Ringelement, integriert ist und/oder die Sonde ein Metallrohr aufweist. Mittels des Ringelements lässt sich das RFID-Element mit Vorteil auf einfache Weise schwingungsgeschützt an der Sonde montieren. Auch die Temperaturleitfähigkeit von Kunststoffen ist in der Regel geringer als die von Metall, so dass das RFID-Element vor den hohen Temperaturen bei einer Dampfsterilisation geschützt wird. Vorzugsweise ummantelt das Ringelement im montierten Zustand die Sonde. Insbesondere ummantelt das Ringelement das Adapterelement, beispielsweise in Zentralrichtung gesehen auf Höhe der ersten Aussparung. Weiterhin ist es vorgesehen, dass das Ringelement eine Auslassung zur Aufnahme des RFID-Elements aufweist. In diese Auslassung lässt sich das RFID-Element platzieren und vorzugsweise stoffschlüssig mit dem Ringelement verbinden. Im an der Sonde montierten Zustand ist dabei eine ringelementseitige Öffnung, über die das RFID-Element in die Auslassung eingeführt wird, der Quelle zugewandt. Mit anderen Worten: das RFID-Element ist auf der im montierten Zustand der Quelle zugewandten Seite des Ringelements angeordnet. Mit der als Metallrohr ausgestalten Sonde ist es in vorteilhafter Weise möglich, Körpersteinfragmente abzusaugen.

Ferner ist es vorstellbar, dass das RFID-Element dabei so ausgerichtet ist, dass seine Signalstärke in eine vom ersten Ende der Sonde abgewandten Richtung maximal ist. Weiterhin ist es vorgesehen, dass das Ringelement, in einer bezogen auf die Zentralachse radialen Richtung gesehen, gegenüber einem äußersten Rand der Sonde, insbesondere des Adapterelements, übersteht. Dadurch ist es möglich, dass das RFID-Element nicht von der metallischen Sonde, insbesondere einem Kragen am Adapterelement, verdeckt ist. Dies trägt ebenfalls zur Funktionalitätssicherung des RFID-Elements bei.

Vorzugsweise weist das in das Ringelement eingelassene RFID-Element einen quadratischen Querschnitt auf und ist kleiner als 5 x 5 mm, bevorzugt kleiner als 4 x 4 mm und besonders bevorzugt kleiner als 3 x 3 mm. Mit einem derart klein dimensionierten Querschnitt, beispielswiese von 2,7 x 2,7 mm, wird mit Vorteil ein vergleichsweises kleines RFID-Element in das Ringelement integriert.

Ferner ist es vorstellbar, dass das Ringelement eine visuell erfassbare Kennzeichnung umfasst, beispielsweise eine bestimmte Farbe, eine bestimmte Außenkontur (z. B. rund, achtechig, neuneckig...) und/oder einen Bar-oder QR-Code. Dadurch lässt sich für den Nutzer auf einfache Weise bereits ohne Nutzung des RFID-Elements erkennen, um welchen Sondentyp es sich handelt. Hierzu lässt sich beispielsweise eine bestimmte Farbe für jeden Sondentyp festlegen. Die einzelnen Sondentypen untescheiden sich beispielsweise hinsichtlich ihrers Einsatzswecks, der Dimensionierung, z. B. in Hinblick auf die Länge des Hohlkörpers und/oder des Durchmessers.

In einer weiteren Ausführungsform der vorliegenden Erfindung ist vorgesehen, dass das Ringelement spielbehaftet unter Ausbildung eines Spalts an der Sonde montierbar ist, wobei eine Spaltbreite des Spalts vorzugsweise einen Wert zwischen 0,05 mm und 0,2 mm, bevorzugt einen Wert zwischen 0,08 mm und 0,13 mm und besonders bevorzugt einen Wert von im Wesentlichen 0,1 mm annimmt. Durch den Spalt wird in vorteilhafter Weise verhindert, dass das Ringelement an der Sonde, insbesondere dem Adapterelement, fest anliegt. Folge ist eine ausreichende Entkopplung von den betriebsbegleitenden Schwingungen der Sonde. Gerade für einen Spaltbreite von 0,1 mm hat sich in vorteilhafter Weise herausgestellt, dass sich nicht nur eine ausreichende Endkopplung realisieren lässt, sondern auch das Ringelement an der Sonde so angeordnet ist, dass es nicht klappert oder sich sogar im Betrieb löst. Insbesondere ist unter spielbehaftet zu verstehen, dass sich im montierten Zustand das Ringelement mit dem Finger leicht verdrehen lässt. Insbesondere bildet sich ein Spalt aus, der sich bezogen auf die Zentralrichtung radial zwischen der Sonde und dem Ringelement erstreckt.

Vorzugsweise ist es vorgesehen, dass ein Spaltmass bzw. Spaltbreite mindestens so bemessen ist, dass es bzw. sie grösser ist, als die durch die Schwingungen verursachten Bewegungen des Ringelements. So lässt sich eine ausreichende Entkopplung gewährleisten. Die durch die Schwingungen induzierten Bewegungen der Sonde im Bereich des Ringelementes haben beispielsweise eine Amplitude unterhalb von 0,1 mm.

Vorzugsweise sind die Sonde und die Quelle mittels eines Adapterelements verbindbar, wobei das Ringelement zur axialen Sicherung zwischen einem Kragen des Adapterelements und einem Vorsprung des Adapterelements anbindbar ist. Dadurch lässt sich mit Vorteil verhindern, dass sich das Ringelement in axialer Richtung gesehen verschiebt und sich so von der Sonde löst. Insbesondere ist dabei der Vorsprung derart dimensioniert, dass sich das Ringelement bei dessen Montage über den Vorsprung ziehen lässt.

Zweckmäßig ist es vorgesehen, dass das System eine Steuervorrichtung umfasst, wobei die Steuervorrichtung abhängig von einer durch das RFID-Element übermittelten Information Parameter an der Ultraschallquelle einstellt. Beispielsweise ist die Steuervorrichtung in ein Tischgerät integriert und umfasst ein Lesegerät bzw. Empfänger, der die Signale vom RFID-Element empfängt. Die empfangenen Informationen kann die Steuervorrichtung dann mit Vorteil dazu nutzen, die für den jeweiligen Sondentyp optimierten Parameter automatisch für die erste und/oder zweite Teilquelle festzulegen. Darüber hinaus ist es vorstellbar, dass die Steuervorrichtung den Betrieb des Systems unterbindet, wenn die angebundene Sonde nicht kompatibel ist mit der Quelle oder seine Lebenszeit (1-Weg-Sonde, 5-fach Sonde) überschritten ist.

Vorzugsweise ist das System derart konfiguriert, dass eine sich zeitlich ändernde Zustandsinformation mittels des RFID-Elements übermittelt wird. Beispielsweise handelt es sich bei der zeitlich ändernden Zustandsinformation um die Anzahl der noch möglichen Verwendungen der Sonde. Hierzu ist es vorgesehen, dass nach jeder Benutzung der Sonde die Informationen über die Anzahl der noch möglichen Verwendungen jeweils um eins reduziert wird. Damit lässt sich mit Vorteil sicherstellen, dass die Sonde nicht zu oft benutzt wird, da sie ansonsten bricht. Vorzugsweise ist die Steuervorrichtung bzw. ein Sender in der Steuervorrichtung derart ausgelegt, entsprechend gespeicherte Informationen im RFID-Element zu ändern bzw. zu überschreiben. Insbesondere ist die Steuervorrichtung derart konfiguriert, dass ein Betrieb des Systems unterbunden wird, wenn die Anzahl der möglichen Verwendungen auf null abgesunken ist. Dies verhindert mit Vorteil die unsachgemäße Nutzung der Sonde und ggf. eine Verletzung des Patienten.

Besonders bevorzugt ist es vorgesehen, dass die Quelle in ein Handgerät integriert ist und vorzugsweise ein Sender, insbesondere eine ringförmige Antenne, für das RFID-Element in das Handgerät eingelassen ist. Insbesondere umfasst der Sender ein Lesegerät und eine Antenne, mit der der Sender einerseits mit dem RFID-Element kommunizieren kann und andererseits mit der Steuervorrichtung zum Bereitstellen und Überschreiben von Informationen, die in der Speichereinrichtung des RFID-Elements gespeichert sind. Mit anderen Worten: ein Teil des RFID-Senders wird in das Handgerät ausgelagert und ist beispielsweise im verbundenen Zustand mit dem RFID-Element funktionsbereit. Über ein Kabel, insbesondere ein Koaxialkabel, kann der Sender dabei Informationen mit dem RDIF-Element austauschen. Durch die Auslagerung der Kommunikationsschnittstelle für das RDIF-Element in das Handgerät kann zum einen auf entsprechende Komponenten in bzw. an der Sonde verzichtet werden und zum anderen lässt sich die Platzierung für eine möglichst störfreie Übertragung von drahtlos übermittelten Signalen optimieren. Beispielsweise ist die ringförmige Antenne im zusammengesetzten Zustand von Quelle und Sonde parallel zum Ringelement, besonders bevorzugt konzentrisch zum Ringelement ausgerichtet. Ferner ist es denkbar, dass die handgerätseitige Antenne, insbesondere ringförmige Antenne, in einem im montierten Zustand der Sonde zugewandten Bereich des Handgeräts angeordnet.

Vorteilhafter Weise ist es vorgesehen, wenn das Ringelement aus einem sterilisierbaren Material, insbesondere Polyphenylensulfon (PPSU), gefertigt ist. Unteren einem sterilisierbaren Material ist insbesondere ein solches zu verstehen, dass sich mittels eines Sterilisationsverfahren sterilisieren lässt, vorzugsweise mit demselben Sterilisationsverfahren, mit dem die Sonde sterilisiert wird, ohne dass das Ringelement durch das Sterilisationsverfahren wesentlich modifiziert wird. Durch die Sterilisation ist es möglich, das Ringelement beispielsweise für die intra-korporale Lithotripsie zu verwenden. Vorstellbar ist es dabei, dass das Ringelement zusammen mit der Sonde sterilisiert wird.

Ein weiterer Gegenstand der vorliegenden Beschreibung ist ein Adapterelement für ein System zum Zertrümmern und/oder Entfernen von Körpersteinen, insbesondere für ein erfindungsgemäßes System, wobei mittels des Adapterelements eine Sonde und eine Quelle über ein Gewinde miteinander verbindbar sind, wobei das Gewinde einen Kunststoffbereich aufweist, wobei der Kunststoffbereich vorzugsweise durch eine Kunststoffbeschichtung und/oder einen Einsatz realisiert ist. Alle für das erfindungsgemäße System beschriebenen Merkmale und deren Vorteile lassen sich sinngemäß ebenfalls auf das Adapterelement übertragen und andersherum.

Gegenüber den aus dem Stand der Technik bekannten Adapterelementen, erweist sich das Ausstatten des Gewindes mit einem Kunststoffbereich insofern als vorteilhaft, als dass dadurch eine gegen Schwingungen stabilere Verbindung zwischen der Quelle und der Sonde realisierbar ist, ohne die ineinandergreifenden Gewinde von Quelle und Sonde zu fest anziehen zu müssen. Ein solches übermäßiges Festziehen birgt nämlich die Gefahr, dass die beiden ineinandergreifenden Gewinde durch die Ultraschallbewegung im Betrieb miteinander verschweißt werden und das System irreversibel geschädigt wird. Daher werden die Sonde und die Quelle über ihre Gewinde vorzugsweise mit einem festgelegten Drehmoment zueinander festgezogen. Als besonders vorteilhaft erweist es sich, dass der Kunststoffbereich adapterelementseitig ausgestaltet ist, da mit jedem Verbinden der Sonde und der Quelle über das Gewinde eine hemmende Wirkung durch den Kunststoffbereich reduziert wird. Insbesondere ist es dabei vorgesehen, dass zum Verbinden die Sonde ein Innengewinde aufweist und die Quelle bzw. ein Handgerät, in die die Quelle integriert ist, ein komplementäres Außengewinde bereitstellt.

Vorzugsweise ist es vorgesehen, dass der Kunststoffbereich derart dimensioniert oder ausgestaltet ist, dass er für eine bestimmte Anzahl an Verbindungen eine hemmende Wirkung sicherstellt, wobei diese Anzahl bevorzugt abgestimmt ist mit der möglichen Anzahl an potentiellen Einsätzen für die Sonde. Weiterhin ist es vorstellbar, dass die sich der Kunststoffbereich nicht über die gesamte Länge des Gewindes erstreckt. Dadurch ist es in vorteilhafter Weise möglich, dass das Außen und- Innengewinde zumindest teilweise leichtgängig verschraubbar sind, insbesondere solange das Außengewinde und das Innengewinde in einen Bereich außerhalb des Kunststoffbereichs ineinandergreifen. Beispielswiese ist der Kunststoffbereich durch eine Beschichtung, insbesondere einzelner Gewindegänge, realisiert. Es ist aber auch vorstellbar, das ein Kunststoffeinsatz derart in das Adapterelement eingelassen ist, dass er in den durch den generellen Verlauf des Innengewindes des Adapterelements aufgespannten Aufnahmebereich bzw. Innenraum hineinragt. Beim ersten Verbinden des Außengewindes der Quelle mit der Sonde wird dann ein Gewindegang in den Einsatz eingeschnitten bzw. eingeformt.

Gemäß einem anderen Aspekt der vorliegenden Erfindung ist ein Verfahren zum Bereitstellen eines System zum Zertrümmern und/oder Entfernen von Körpersteinen, insbesondere eines erfindungsgemäßen Systems, vorgesehen, wobei die Sonde und die Quelle miteinander verbunden werden, wobei vorzugsweise die Sonde und die Quelle mit einem festgelegten Drehmoment aneinander angeschraubt werden und/oder von einem RFID-Element Signale ausgesendet werden. Alle für das erfindungsgemäße System beschriebenen Merkmale und deren Vorteile lassen sich sinngemäß ebenfalls auf das Verfahren übertragen und andersherum. Vorzugsweise werden die Sonde und die Quelle mittels eines Drehmomentschlüssels miteinander verschraubt.

Weitere Vorteile und Merkmale ergeben sich aus der nachfolgenden Beschreibung bevorzugter Ausführungsformen des erfindungsgemäßen Gegenstands mit Bezug auf die beigefügten Figuren. Einzelne Merkmale der einzelnen Ausführungsform können dabei im Rahmen der Erfindung miteinander kombiniert werden.

Es zeigt:
- **Fig.1:**: ein System zum Zertrümmern und/oder Entfernen von Körpersteinen gemäß einer ersten bevorzugten Ausführungsform der vorliegenden Erfindung,
- **Fig.2 und 2b:**: Sonde für ein System gemäß der ersten bevorzugten Ausführungsform,
- **Fig.3a und 3b:**: Detailansicht eines Adapterelements mit einem Ringelement für ein System der bevorzugten Ausführungsform der vorliegenden Erfindung, in einer Schnittansicht (3a) und einer Draufsicht (3b),
- **Fig.4:**: Explosionsdarstellung der Detailansicht aus den Figuren 3a und 3b,
- **Fig.5:**: ein Adapterelement der Sonde aus den Figuren 2 bis 4,
- **Fig. 6:**: ein Ringelement für die Sonde aus den Figuren 2 bis 5, und
- **Fig.7.**: ein Adapterelement für eine Sonde gemäß einer zweiten bevorzugten Ausführungsform der vorliegenden Erfindung.

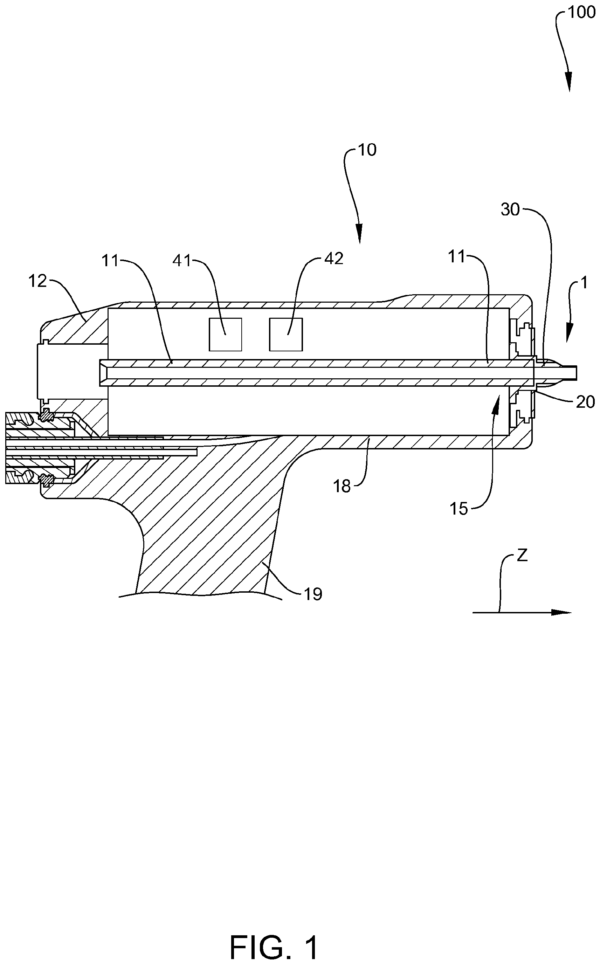

In der **Figur 1** ist schematisch ein System 100 zum Zertrümmern und/oder Entfernen von Körpersteinen illustriert. Beispielsweise handelt es sich bei den Körpersteinen um Nieren- oder Harnsteine, die sich mittels des Systems 100 zertrümmern lassen. Wesentliche Bestandteile eines solchen Systems 100 sind eine Stoßwellen und/oder Ultraschallwellen veranlassende Quelle 10 sowie eine Sonde 1. Dabei ist es insbesondere vorgesehen, dass die von der Quelle 10 ausgehenden Stoßwellen und/oder Ultraschallwellen auf die Sonde 1 übertragen werden, wodurch ein im Betrieb dem Körperstein zugewandtes erstes proximales Ende 21 der Sonde 1, das von der Quelle 10 abgewandt ist, ein Zertrümmern des Körpersteins oder von Teilen des Körpersteins bedingt.

Zum Übertragen der Stoßwellen und/oder der Ultraschellwellen sind die Quelle 10 und die Sonde 1 über eine Schnittstelle 15 miteinander lösbar verbunden. In dem in Figur 1 dargestellten Ausführungsbeispiel ist es vorgesehen, dass die Quelle 10 in ein Handgerät 12, insbesondere mit einem Projektilbereich 18 und einem Griffbereich 19, integriert ist und zur Übertragung der Stoßwellen ein Projektil 11 und zur Übertragung der Ultraschallwellen Piezoelemente im Projektilbereich 18 vorgesehen sind, wobei das Projektil 11 mittels einer ersten Teilquelle 41 zu einer Stoßbewegung und die Sonde 1 mittels einer zweiten Teilquelle 42 zu einer Ultraschallbewegung angetrieben wird. Im betriebsbereiten Zustand sind das Projektil 11 und ein dem Projektil 11 zugewandtes zweites distales Ende 22 der Sonde 1 zueinander fluchtend angeordnet und das Projektil 11 wirkt zur Übertragung der Stoßwellen auf das zweite Ende 22 der Sonde 1, vorzugsweise schlagend bzw. hämmernd, ein. Die zweite Teilquelle 42 besteht aus Piezoelementen, die ringförmig um den Projektilkanal angeordnet sind und direkt Ultraschallwellen in die Sonde an der Schnittstelle 15 einkoppeln. Als besonders vorteilhaft hat es sich erwiesen, wenn die Stoßwellen von den vorzugsweise gegenüber den Stoßwellen höherfrequenten Ultraschallwellen überlagert werden.

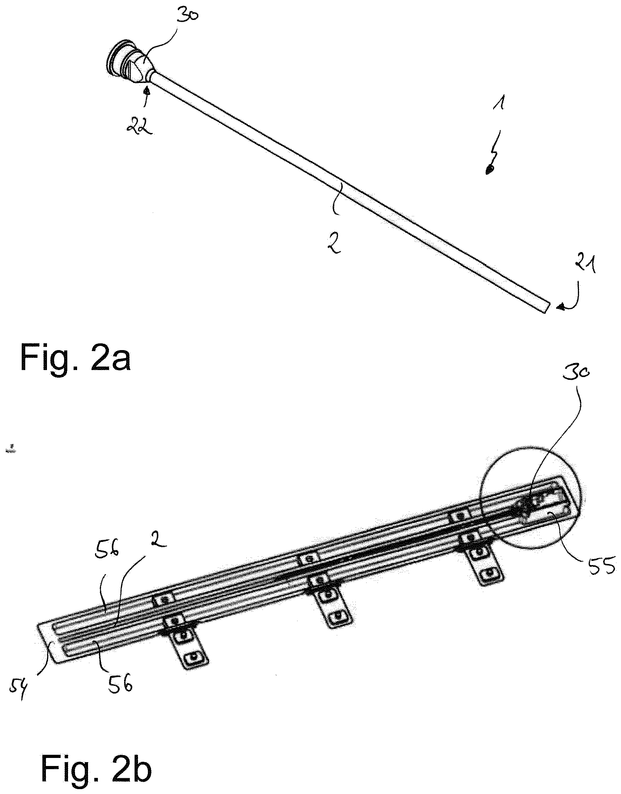

Weiterhin ist es vorgesehen, dass die Sonde 1 nadelförmig ausgestaltet ist, wie es in **Figur 2a** zu erkennen ist. Die **Figur 2b** zeigt die Sonde 1 in einem verpackten Zustand, vorzugsweise steril verpackten Zustand. Die nadelförmig ausgestaltete Sonde 1 nimmt dabei mit Vorteil die am zweiten Ende 22 auf die Sonde 1 eingeleiteten Stoß- und/oder Schallwellen auf und leitet sie zum ersten Ende 21 der Sonde 1. Das am Körperstein platzierte erste Ende 21 der Sonde 1 bedingt dann im Betrieb ein Zertrümmern des Körpersteins. Darüber hinaus ist es mit Vorteil vorgesehen, dass die nadelförmige Sonde 1 hohl ist, um über die Sonde 1, insbesondere den hierfür vorgesehenen Hohlbereich 25 in der Sonde 1, die zertrümmerten bzw. zerkleinerten Teile der Körpersteine abzusaugen bzw. zu entfernen. Hierzu wird beispielsweise ein Unterdruck im Handgerät 12 erzeugt, der das entsprechende Absaugen bedingt.

Ferner ist es vorgesehen, dass die Sonde 1 einen die Nadelform im Wesentlichen bestimmenden Hohlkörper 2 und an ihrem zweiten Ende 22 ein Adapterelement 30 aufweist. In dem in Figur 2b gezeigten verpackten Zustand ist das Adapterelement 30 in einem gehäuseartigen Schutzkörper 55 und der Hohlkörper 2 zwischen zwei Schutzstegen 56 gelagert. Die Sonde 1 wird dabei auf eine Träger 54 angeordnet und vorzugsweise von eine Hülle, insbesondere einer Plastikhülle, umschlossen bzw. ummantelt.

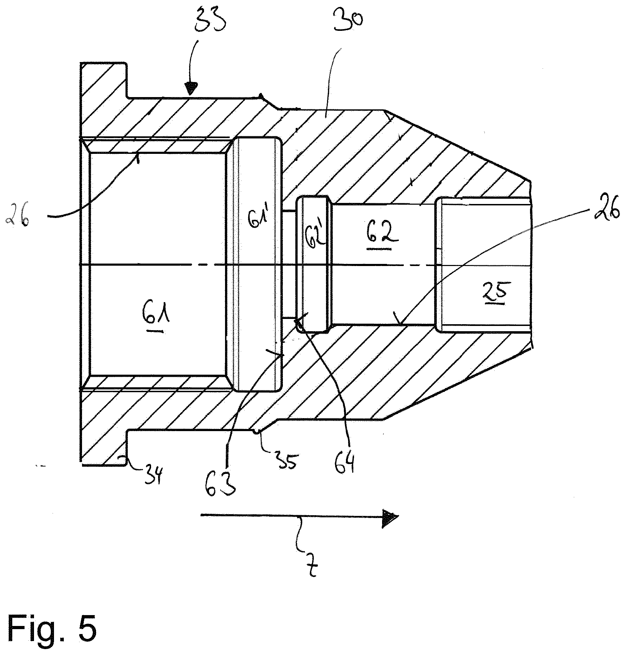

**Figur 5** zeigt das Adapterelement 30 schematisch in einer Schnittansicht. Das Adapterelement 30 ist hülsenförmig ausgestaltet, wobei ein Verlauf einer Innenseite 26 des Adapterelements 30 in einer Zentralrichtung Z gesehen gestuft ausgestaltet ist. Dabei verläuft die Zentralrichtung Z im Wesentlichen parallel zu einer Richtung entlang der Richtung der Schallwellen und/oder Stoßwellen die auf die Sonde 1 übertragen werden. Durch den gestuften Verlauf an der Innenseite 26 des Adapterelements 30 bilden sich vorzugsweise gegenüberliegend eine erste Aussparung 61 und eine zweite Aussparung 62 aus. Im Betrieb trifft das Projektil 11 in die erste Aussparung 61 und der die Nadelform im Wesentlichen bestimmende Hohlkörper 2 ist innerhalb der zweiten Aussparung 62 angeordnet. Die erste Aussparung 61 wird in Zentralrichtung Z gesehen durch eine erste Abschlussfläche 63 und die zweite Aussparung 62 in entgegengesetzter Richtung durch eine zweite Abschlussfläche 64 begrenzt. Mit der ersten Abschlussfläche 63 verjüngt der gestufte Verlauf an der Innenseite 26 dabei in Zentralrichtung Z gesehen derart, dass ein Innendurchmesser in diesem Bereich kleiner ist als der Außendurchmesser des Projektils 11, während mit der zweiten Abschlussfläche 64 der gestufte Verlauf derart verjüngt, das in diesem Bereich der Innendurchmesser kleiner ist als ein Außendurchmesser des Hohlkörpers 2. Dabei ist es vorgesehen, dass in Zentralrichtung Z gesehen die erste Aussparung 61 sich über eine erste Länge L1 erstreckt und die zweite Aussparung 62 über eine zweite Länge L2 erstreckt, wobei das Verhältnis zwischen der ersten Länge L1 und der zweiten Länge L2 einen Wert zwischen 0,75 und 0,9, bevorzugt zwischen 0,78 und 0,85 und besonders bevorzugt zwischen 0,79 und 0,82 annimmt. Weiterhin ist es vorgesehen, dass in Zentralrichtung Z gesehen ein senkrecht zur Zentralrichtung Z verlaufender Querschnitt in einem an die erste bzw. die zweite Abschlussfläche angrenzenden ersten Teilbereich 61' der ersten Aussparung 61 bzw. zweiten Teilbereich 62' der zweiten Aussparung 62 partiell aufgeweitet ist, insbesondere größer ist als ein Außendurchmesser des Projktils 11 bzw. des Hohlkörpers 2.

Weiterhin ist es vorgesehen, dass die Sonde 1, vorzugsweise auch das Adapterelement 30, aus einem Metall, insbesondere einem Edelstahl gefertigt ist. Außerdem umfasst das Adapterelement 30 an seiner Außenseite einen weiteren Griffbereich 31, beispielsweise in Form zweier im Wesentlichen zueinander parallel bzw. zueinander leicht geneigten ebenen Flächen 38.

Bei den Sonden 1 handelt es sich um einen Verschleißgegenstand, der nach einer begrenzten Anzahl an Einsätzen nicht mehr verwendet werden kann. Daher sind die Quelle 10 und die Sonde 1 so gestaltet, dass sie sich lösbar miteinander verbinden bzw. austauschen lassen, d. h. sie sind reversibel verbindbar. Dadurch ist es auch möglich, verschiedene Sondentypen mit derselben Quelle 10 zu verwenden.

Um den unsachgemäßen Gebrauch der Sonden 1 zu verhindern, ist es vorgesehen, dass die Sonde 1 ein Ident- bzw. RFID-Element 5 umfasst. Mittels des RFID-Elements 5 ist es mit Vorteil möglich, die an die Quelle 10 angebundene Sonde 1, insbesondere in Hinblick auf ihren Zustand und/oder Sondentyp, zu identifizieren. Dadurch lässt sich verhindern, dass das System 10 mit einem ungeeigneten Sondentyp betrieben wird. Außerdem ist es bevorzugt vorgesehen, dass Parameter für den Betrieb der Quelle, insbesondere der ersten Teilquelle 41 und/oder der zweiten Teilquelle 42, an den jeweiligen aktuell verwendeten Sondentyp angepasst werden. Hierzu kommuniziert das RFID-Element 5 mit einer Steuervorrichtung, die bspw. in eine Tischeinheit integriert ist, und übermittelt der Steuervorrichtung die benötigte Information über den aktuell an die Quelle 10 angebundenen Sondentyp. Vorzugsweise werden anschließend die gewünschten Parameter eingestellt oder ein Nutzer kann aus eine Liste von für den Sondentyp bevorzugten Parametern wählen. Weiterhin ist es vorgesehen, dass ein Sender, beispielsweise eine Antenne und/oder ein Lesegerät, in das Handgerät 12 integriert ist, wobei der Sender im betriebsbereiten Zustand des Systems 100 (d. h. wenn die Sonde 1 und Quelle 10 miteinander verbunden sind) mit dem RFID-Element 5 in einer Kommunikationsverbindung steht, beispielsweise über ein Kabel, insbesondere einem Koaxialkabel.

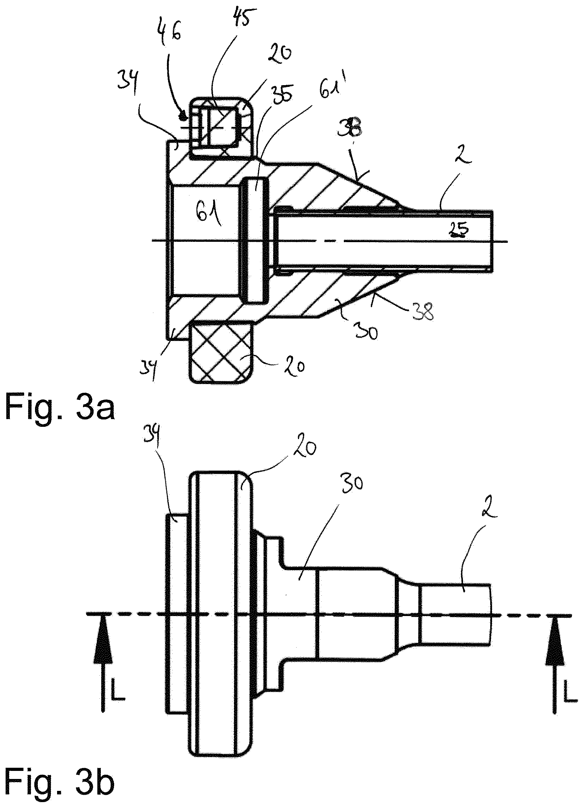

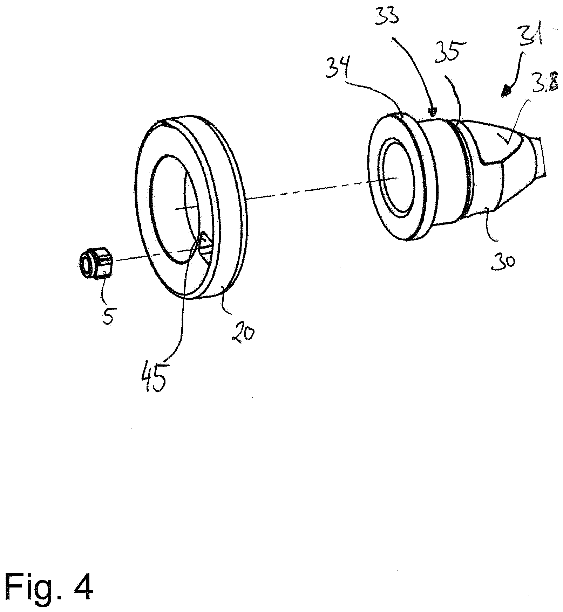

Allerdings beeinträchtigen die auf die RFID-Elemente 5 wirkenden Ultraschallschwingungen und das Material der Sonde 1 die Funktionalität des RFID Elements 5. Um die Funktionalität der Sonde 1 im Betrieb zu verbessern, ist es mit Vorteil vorgesehen, das RFID-Element 5 schallgeschützt an der Sonde 1 anzuordnen. Hierzu wird das RFID-Element 5 vorzugsweise in ein Ringelement 20, insbesondere in ein aus Kunststoff gefertigtes Ringelement 20, integriert. Das Ringelement 20 wird wiederum an der Sonde 1 montiert, insbesondere an einer Außenseite der Sonde und mit Vorteil in einem Anschlussbereich 33 an eine Außenseite des Adapterelements 30. Die **Figuren 3a und 3b** zeigen das Adapterelement 30 mit montiertem Ringelement 20 in einer Schnittansicht (Figur 3a) und einer Draufsicht (Figur 3b) und **Figur 4** stellt eine Explosionsdarstellung von Adapterelement 30, Ringelement 20 und RFID-Element 5 dar.

Dabei wird das Ringelement 20 bevorzugt derart spielbehaftet an die Sonde 1 montiert, dass eine Übertragung von Ultraschallschwingungen auf das Ringelement 20 reduziert ist und gleichzeitig das Ringelement 20 einen ausreichend festen Sitz an der Sonde 1 vorweisen kann. In einem solchen spielbehafteten Zustand befindet sich das Ringelement 20 beispielsweise, wenn sich das Ringelement 20 leicht mit den Fingern drehen lässt. Ferner ist es vorgesehen, dass das Material, aus dem das Ringelement 20 gefertigt, sterilisierbar ist. Als besonders vorteilhaft hat sich hierbei Polyphenylensulfon (PPSU) als Material herausgestellt.

In dem dargestellten Ausführungsbeispiel ist das Ringelement 20 am Adapterelement 30 angeordnet, insbesondere in Zentralrichtung Z gesehen auf Höhe der ersten Aussparung 61. Zur Lagerung des Ringelements 20 weist das Adapterelement 30, an seinem im montierten Zustand der Sonde der Quelle zugewandten Ende einen umlaufenden Kragen 34 auf. Dieser Kragen 34 bildet mit Vorteil einen Anschlag für das Ringelement 20. In Zentralrichtung Z gesehen ist versetzt zum Kragen 34 an der Außenseite des Adapterelements eine Vorsprung 35 bzw. eine Nase vorgesehen. Vorzugsweise ist das Ringelement 20 zur axialen Sicherung, d. h. zur Vermeidung eines Verrutschens des Ringelements 20 in axialer Richtung (parallel zur Zentralrichtung), zwischen dem Vorsprung 35 und dem Kragen 34 angeordnet.

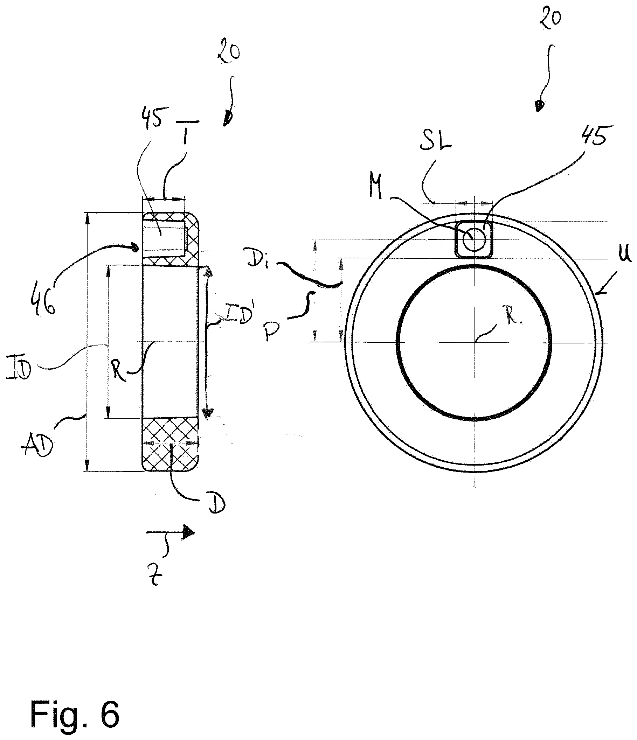

In **Figur 6** ist das Ringelement 20 mit einem Innendurchmesser ID und einem Außendurchmesser AD im Detail dargestellt. Beispielsweise nimmt der Außendurchmesser AD einen Wert zwischen 15 und 20 mm, besonders bevorzugt von im Wesentlichen 18,6 mm an, während der Innendurchmesser einen Wert zwischen 10 und 13 mm annimmt, bevorzugt von 11 mm. Weiterhin ist es vorstellbar, dass der Innendurchmesser ID in einer parallel zur Rotationssymmetrieachse R, zu der der äußere Umfang U des Ringelements 20 vorzugsweise vollständig rotationssymmetrisch ist, verlaufenden Richtung gesehen kleiner wird, insbesondere stetig kleiner wird. Beispielsweise weist der Innendurchmesser ID an einer Stirnseite des Ringelements 11 mm und auf der gegenüberliegenden Stirnseite des Ringelements 20 einen weiteren Innendurchmesser ID' von 10,8 mm auf.

Zur Aufnahme des RFID-Elements 5 ist eine Auslassung 45 vorgesehen. Bezogen auf die Rotationssymmetrieachse R beginnt die Auslassung 45 in radialer Richtung gesehen in einer Distanz Di und ist mit seiner Mitte M von der Rotationssymmetrieachse R um einen Positionsabstand P entfernt angeordnet. Der Positionsabstand P ist vorzugsweise so dimensioniert, dass die Auslassung 45 in radialer Richtung gesehen mittig zwischen Innendurchmesser ID und Außendurchmesser AD angeordnet ist. Insbesondere ist das Ringelement 5 im montierten Zustand derart ausgerichtet, dass eine ringelementseitige Öffnung 46, über die das RFID-Element 5 in die Auslassung 45 eingelassen werden kann, dem Kragen 34 des Adapterelements 30 bzw. der Quelle 1 zugewandt ist. Ferner nimmt ein Verhältnis zwischen einer in Zentralrichtung Z bemessenen Tiefe T der Auslassung 45 zu einer in derselben Richtung bemessenen Dicke D des Ringelements 20 einen Wert zwischen 0,75 und 0,98, bevorzugt zwischen 0,8 und 0,95 und besonders bevorzugt zwischen 0,85 und 0,94 an. Weiterhin ist es vorgesehen, dass im montierten Zustand bezogen auf die Zentralrichtung Z in radialer Richtung gesehen, das Ringelement 20, insbesondere dessen äußerer Umfang U, gegenüber dem Kragen 34 des Adapterelements 30 vorsteht. Insbesondere steht das Ringelement 20 derart weit vor, dass der Öffnung 46 der Auslassung 45 bzw. ein Teil der Auslassung 45 frei liegt (siehe Figur 3a). Vorzugsweise ist die Auslassung 45 durch den Kragen 34 weniger als 20 %, bevorzugt weniger als 15 % und besonders bevorzugt weniger als 10 % bedeckt. Dadurch bleibt mit Vorteil das in das Ringelement 20 eingelassene RFID-Element 5 zugänglich bzw. es kann eine zuverlässige Kommunikation mit dem RFID-Element 5 zum Informationsaustausch sichergestellt werden.

Zum Fixieren des RFID-Elements 5 in der Auslassung 5 ist eine stoffschlüssige Verbindung, beispielsweise mittels eines Klebstoffs, vorgesehen. Dabei ist die Auslassung 5 vorzugsweise nur partiell mit dem Klebstoff zu füllen bzw. gefüllt. Weiterhin ist es vorgesehen, dass die Auslassung quadratisch gestaltet ist, insbesondere mit einer Seitenlänge SL. Weiterhin ist es vorzugsweise vorgesehen, dass ein Verhältnis der Dicke D des Ringelements 20 zur ersten Länge L1 einen Wert zwischen 0,5 und 0,9, bevorzugt zwischen 0,55 und 0,8 und besonders bevorzugt zwischen 0,63 und 0,73 annimmt.

Das Anbinden der Sonde 1 an die Quelle 10 erfolgt bevorzugt über ein Gewinde 71. Beispielsweise weist das Adapterelement 30, insbesondere in Zentralrichtung Z gesehen auf Höhe des Kragens 34, an seiner Innenseite 26 ein Innengewinde auf. Dabei ist beim Anschrauben des Adapterelements 30 an die Quelle 10 darauf zu achten, dass die Adapterelement 30 nicht zu fest angezogen wird, da sonst andernfalls die im Betrieb auftretenden Schwingungen das Adapterelement 30 stoffschlüssig mit der Quelle 10, insbesondere deren Gewinde, verbindet und so die Quelle 10 bzw. das Handgerät 12 zerstört wird. Daher hat es sich als vorteilhaft erwiesen, die Sonde 1 mit einem festgelegten Drehmoment, vorzugsweise mittels eines Drehmomentschlüssels, kontrolliert an die Quelle 10 anzubinden.

Um trotz des erforderlichen reduzierten Drehmoments, mit dem die Sonde 1 an die Quelle 10 gebunden wird, ein Lösen des Adapterelements 30 von der Sonde 1 wegen der im Betrieb auftretenden Schwingungen entgegenzuwirken, ist es in der dargestellten Ausführungsform vorgesehen, dass das Gewinde 71 beschichtet ist, insbesondere mit einer Kunststoffbeschichtung beschichtet ist. Dabei sind vorzugsweise nur einzelne Gewindegänge oder alle Gewindegänge des Gewindes 71 mit der Kunststoffbeschichtung bedeckt. Insbesondere sind nur die Gewindegänge beschichtet, in die das handgerätseitige Gegengewinde bzw. Außengewinde beim Verbinden zuletzt eingreift. Dadurch wird das anfängliche Anschrauben mit Vorteil erleichtert und die Beschichtung auf den letzten Gewindegängen stabilisiert die lösbare Verbindung zwischen der Sonde 1 und der Quelle 10. Beispielsweise sind nur die beiden letzten Gewindegänge mit der Kunststoffbeschichtung versehen. Vorzugsweise nimmt das Verhältnis der Zahl der Gewindegänge mit Beschichtungen zu einer Zahl der Gewindegänge ohne Beschichtung einen Wert kleiner als 1, bevorzugt kleiner als 0,5 und besonders bevorzugt kleiner als 0,25 an. Dabei hat es sich als vorteilhaft erwiesen, dass sich die Kunststoffbeschichtung beim wiederholten Aufsetzen und Abnehmen der Sonde 1 abnutzt. Insbesondere ist die Kunststoffbeschichtung, beispielsweise durch geeignete Wahl des Materials, der Anzahl der beschichteten Gewindegänge und/oder der Schichtdicke, derart gestaltet, dass sich so die Anzahl der möglichen Anschraubvorgänge festlegen lässt. Insbesondere ist diese Anzahl an möglichen Anschraubvorgängen abgestimmte mit der maximalen Anzahl an möglichen Einsetzten für die Sonde 1.

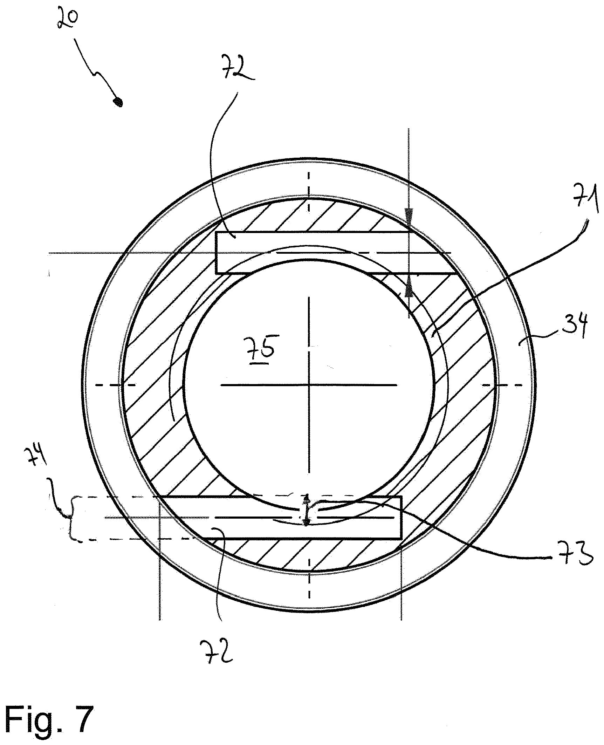

In **Figur 7** ist ein Adapterelement 30 für ein System 1 gemäß einer zweiten bevorzugten Ausführungsform der vorliegenden Erfindung dargestellt. Dabei unterscheidet sich das Adapterelement 30 dahingehend von dem aus der Figur 5 im Wesentlichen nur hinsichtlich des Gewindes 71, mit dem die Sonde 1 an die Quelle 10 gebunden wird. Statt einzelne Gewindegänge zu beschichteten, ist es hier vorgesehen, dass ein oder mehrere stiftartige Einsätze 72, vorzugsweise Kunststoffeinsätze, in das Adapterelement 30 eingelassen sind. Insbesondere wird hierbei das Adapterelement 30 mit einer Bohrung versehen, die bevorzugt tangential zum Innengewinde verläuft und in die sich der stiftartige Einsatz 72, insbesondere ein Kunststoffeinsatz, einschieben lässt. In einem eingesetzten bzw. eingeschobenen Zustand ragt der Einsatz 72 vor dem ersten Verbinden mit der Quelle 1 in einen Innenraum 75 des Adapterelements 30 hinein bzw. steht gegenüber dem Gewinde 71 in radialer Richtung gesehen nach innen vor. Durch eine geeignete Materialwahl für den Einsatz 72 ist es dann möglich, dass bei einem ersten Verbinden der Sonde 1 mit der Quelle 10 das Gewinde der Quelle, insbesondere ein Außengewinde der Quelle, ein Gewinde in den im Adapterelement eingelassenen Einsatz 72 hineinschneidet. Es hat sich mit Vorteil herausgestellt, dass der Einsatz 72 das Gewinde der Quelle 10 hemmt und so ein Lösen der Verbindung zwischen der Quelle 10 und der Sonde 1 im Betrieb wirkungsvoll verhindert. Bevorzugt lässt sich die Sonde 1 mit dem oder den Einsätzen bis zu fünf Mal anschrauben.

Sofern zwei Einsätze 72 vorgesehen sind, sind diese im Wesentlichen parallel zueinander ausgerichtet. Weiterhin ist es vorgesehen, dass der Einsatz derart dimensioniert ist, dass im angeschraubten Zustand in radialer Richtung gesehen ein zur Bildung eines Teils des Gewinde eingeschnittene Abschnitt 73 größer ist als eine Breite 74 des stiftartigen Einsatzes 72. Insbesondere nimmt ein Verhältnis des zum Gewinde gehörenden Abschnitts 73 zu der Breite des Einsatzes 72 einen Wert zwischen 0,3 und 0,75, bevorzugt zwischen 0,4 und 0,6 und besonders bevorzugt zwischen 0,45 und 0,55 an.

### Bezugszeichenliste:

- 1: Sonde
- 2: Hohlkörper
- 5: Ident-Element
- 10: Quelle
- 11: Projektil
- 15: Schnittstelle
- 18: Griffbereich
- 19: Projektilbereich
- 20: Ringelement
- 21: erstes Ende
- 22: zweites Ende
- 25: Hohlbereich
- 26: Innenseite
- 30: Adapterelement
- 31: weiterer Griffbereich
- 33: Anschlussbereich
- 34: Kragen
- 35: Vorsprung
- 38: geneigte Fläche
- 41: erste Teilquelle
- 42: zweite Teilquelle
- 45: Auslassung
- 46: Öffnung
- 54: Träger
- 55: Schutzgehäuse
- 56: Schutzsteg
- 61: erste Aussparung
- 61': erster Teilbereich
- 62: zweite Aussparung
- 62': zweiter Teilbereich
- 63: erste Abschlussfläche
- 64: zweite Abschlussfläche
- 71: Gewinde
- 72: Einsatz
- 73: Abschnitt
- 74: Breite
- 75: Innenraum
- 100: System
- AD: Außendurchmesser
- ID: Innendurchmesser
- D: Dicke
- Di: Distanz
- L1: erste Länge
- L2: zweite Länge
- M: Mitte
- P: Position
- R: Rotationssymmetrieachse
- SL: Seitenlänge
- T: Tiefe
- U: Umfang
- Z: Zentralrichtung

## Patentansprüche

1. System (100) zum Zertrümmern und/oder Entfernen von Körpersteinen, umfassend:
- eine Stoßwellen und/oder Ultraschallwellen veranlassende Quelle (10) und
- eine Sonde (1),
wobei die Quelle (10) und die Sonde (1) über eine Schnittstelle (15) zur Übertragung der Stoßwellen und/oder Ultraschallwellen auf die Sonde (1) reversibel verbindbar sind und die Sonde (1) ein Ident-Element (5) zur Identifikation der Sonde (1) umfasst, **dadurch gekennzeichnet, dass** das Ident-Element (5) schwingungsgeschützt in oder an der Sonde (1) angeordnet ist, so dass das Ident-Element (5) von der Sonde (1) entkoppelt ist.

2. System (100) gemäß Anspruch 1, wobei das Ident-Element (5) in ein Ringelement (20), insbesondere in ein aus Kunststoff gefertigtes Ringelement (20), integriert ist und/oder die Sonde (1) ein Metallrohr aufweist.

3. System (100) gemäß Anspruch 2, wobei das Ringelement (20) unter Ausbildung eines Spalts spielbehaftet an der Sonde (1) montierbar ist, wobei eine Spaltbreite des Spalts vorzugsweise einen Wert zwischen 0,05 mm und 0,2 mm, bevorzugt einen Wert zwischen 0,08 und 0,13 und besonders bevorzugt einen Wert von im Wesentlichen 0,1 mm annimmt

4. System (100) gemäß Anspruch 2 oder 3, wobei die Sonde (1) und die Quelle (10) mittels eines Adapterelements (30) verbindbar sind, wobei das Ringelement (20) zur axialen Sicherung zwischen einem Kragen (34) des Adapterelements (30) und einem Vorsprung (35) des Adapterelements (30) anbindbar ist.

5. System (100) gemäß einem der vorhergehenden Ansprüche, wobei das System (100) eine Steuervorrichtung umfasst, wobei die Steuervorrichtung abhängig von einer durch das Ident-Element (5) übermittelten Information Parameter an der Quelle (10) einstellt, insbesondere automatisch einstellt.

6. System (100) gemäß einem der vorhergehenden Ansprüche, wobei das System (100) derart konfiguriert ist, dass eine sich zeitlich ändernde Zustandsinformation mittels des Ident-Elements (5) übermittelt wird.

7. System (100) gemäß einem der vorhergehenden Ansprüche, wobei die Quelle (10) in ein Handgerät (12) integriert ist und ein Sender, insbesondere eine ringförmige Antenne, für das Ident-Element (5) in das Handgerät (12) eingelassen ist.

8. System (100) gemäß einem der Ansprüche 2 bis 7, wobei das Ringelement (20) aus einem sterilisierbaren Material, insbesondere Polyphenylensulfon (PPSU), gefertigt ist.

9. Verfahren zum Bereitstellen eines Systems (100) zum Zertrümmern und/oder Entfernen von Körpersteinen, insbesondere gemäß einem der Ansprüche 1 bis 8, wobei eine Sonde (1) und eine Stoßwellen und/oder Ultraschallwellen veranlassende Quelle (10) miteinander verbunden werden, wobei die Sonde (1) ein Ident-Element (5) zur Identifikation der Sonde (1) umfasst und wobei von einem Ident-Element (5) Signale ausgesendet werden, und wobei das Ident-Element (5) schwingungsgeschützt in oder an der Sonde (1) angeordnet ist, so dass das Ident-Element (5) von der Sonde (1) entkoppelt ist.

## Claims

1. A system (100) for crushing and/or removing body stones, comprising:
- a source (10) inducing shock waves and/or ultrasonic waves
- and
- a probe (1),
wherein the source (10) and the probe (1) are reversibly connectable via an interface (15) for transmitting the shock waves and/or ultrasonic waves to the probe (1), and the probe (1) comprises an identification element (5) for identifying the probe (1),
**characterized in that**
the identification element (5) is arranged in or on the probe (1) to be vibrationally protected, so that the identification element (5) is decoupled from the probe (1).

2. The system (100) according to claim 1, wherein the identification element (5) is integrated in a ring element (20), especially in a ring element (20) made of plastic, and/or the probe (1) includes a metal tube.

3. The system (100) according to claim 2, wherein the ring element (20) can be mounted on the probe (1) with play to form a gap, wherein a gap width of the gap preferably assumes a value between 0.05 mm and 0.2 mm, preferably a value between 0.08 and 0.13 and particularly preferably a value of substantially 0.1 mm.

4. The system (100) according to claims 2 or 3, wherein the probe (1) and the source (10) are connectable using an adapter element (30), wherein the ring element (20) is connectable between a collar (34) of the adapter element (30) and a projection (35) of the adapter element (30) to be axially secured.

5. The system (100) according to one of the preceding claims, wherein the system (100) comprises a control device, wherein the control device adjusts, in particular automatically adjusts parameters at the source (1U) depending on information transmitted by the identification element (5).

6. The system (100) according to one of the preceding claims, wherein the system (100) is configured such that a time-varying status information is transmitted using the identification element (5).

7. The system (100) according to one of the preceding claims, wherein the source (10) is integrated in a handheld device (12) and a transmitter, especially an annular antenna, for the identification element (5) is embedded in the handheld device (12).

8. The system (100) according to one of claims 2 to 7, wherein the ring element (20) is made of a sterilizable material, especially polyphenylene sulfone (PPSU).

9. A method of providing a system (100) for crushing and/or removing body stones, especially according to one of claims 1 to 8, wherein a probe (1) and a source (10) inducing shock waves and/or ultrasonic waves are connected to each other, wherein the probe (1) comprises an identification element (5) for identifying the probe (1) and wherein signals are emitted from an identification element (5), and wherein the identification element (5) is arranged in or on the probe (1) to be vibrationally protected so that the identification element (5) is decoupled from the probe (1).

## Revendications

1. Système (100) pour fragmenter et/ou enlever des calculs du corps, comprenant :
- une source (10) d'induction d'ondes de choc et/ou d'ondes ultrasonores et
- une sonde (1),
dans lequel la source (10) et la sonde (1) peuvent être connectées de manière réversible via une interface (15) pour transmettre les ondes de choc et/ou les ondes ultrasonores à la sonde (1), et la sonde (1) comprend un élément d'identification (5) pour identifier la sonde (1),
**caractérisé en ce que**
l'élément d'identification (5) est disposé dans ou sur la sonde (1) en étant protégé vis-à-vis des vibrations, de sorte que l'élément d'identification (5) est découplé de la sonde (1).

2. Système (100) selon la revendication 1,
dans lequel l'élément d'identification (5) est intégré dans un élément annulaire (20), en particulier dans un élément annulaire (20) fabriqué en matière plastique, et/ou la sonde (1) présente un tube métallique.

3. Système (100) selon la revendication 2,
dans lequel l'élément annulaire (20) peut être monté avec jeu sur la sonde (1) en formant un interstice, la largeur de l'interstice prenant de préférence une valeur comprise entre 0,05 mm et 0,2 mm, de préférence une valeur comprise entre 0,08 et 0,13 et de manière particulièrement préférée une valeur sensiblement de 0,1 mm.

4. Système (100) selon la revendication 2 ou 3,
dans lequel la sonde (1) et la source (10) peuvent être connectées au moyen d'un élément adaptateur (30), l'élément annulaire (20) pouvant être monté entre un collet (34) de l'élément adaptateur (30) et une saillie (35) de l'élément adaptateur (30), afin de le bloquer axialement.

5. Système (100) selon l'une des revendications précédentes,
dans lequel le système (100) comprend un dispositif de commande, le dispositif de commande ajustant, en particulier automatiquement, des paramètres au niveau de la source (10) en fonction d'une information transmise par l'élément d'identification (5).

6. Système (100) selon l'une des revendications précédentes,
dans lequel le système (100) est configuré de telle sorte qu'une information d'état variant dans le temps est transmise au moyen de l'élément d'identification (5).

7. Système (100) selon l'une des revendications précédentes,
dans lequel la source (10) est intégrée dans un appareil à main (12), et un émetteur, en particulier une antenne annulaire, pour l'élément d'identification (5) est encastré(e) dans l'appareil à main (12).

8. Système (100) selon l'une des revendications 2 à 7,
dans lequel l'élément annulaire (20) est réalisé en un matériau stérilisable, en particulier en polyphénylsulfone (PPSU).

9. Procédé pour fournir un système (100) pour fragmenter et/ou enlever des calculs du corps, en particulier selon l'une des revendications 1 à 8, dans lequel une sonde (1) et une source (10) d'induction d'ondes de choc et/ou d'ondes ultrasonores sont connectées l'une à l'autre, la sonde (1) comprenant un élément d'identification (5) pour identifier la sonde (1), et des signaux étant émis d'un élément d'identification (5), l'élément d'identification (5) étant disposé dans ou sur la sonde (1) en étant protégé vis-à-vis des vibrations, de sorte que l'élément d'identification (5) est découplé de la sonde (1).
